# EUROPEAN PATENT SPECIFICATION

(11) **EP 2 568 867 B1**
(45) Date of publication and mention of the grant of the patent: **06.11.2019**
(21) Application number: 11781025.9
(22) Date of filing: 29.04.2011
(51) Int. Cl.: A61B 1/04

(54) **LASER VIDEO ENDOSCOPE**
LASERVIDEO-ENDOSKOP
ENDOSCOPE LASER VIDÉO

(30) Priority: 13.05.2010 US 779214
(43) Date of publication of application: 20.03.2013
(73) Proprietor: Beaver-Visitec International, Inc., Waltham, MA 02452 (US)
(72) Inventor: URAM, Martin, New Jersey 07739 (US)
(74) Representative: Lloyd, Robin
(86) International application number: PCT/US2011/034464
(87) International publication number: WO 2011/142989

(56) References cited:
- EP-A1- 0 512 592
- WO-A1-98/49929
- US-A- 4 807 594
- US-A- 5 121 740
- US-A- 5 788 628
- US-A- 6 080 101
- US-A1- 2006 025 655
- US-A1- 2007 213 586
- US-B1- 6 997 868

## Description

### Background Of The Invention

This invention relates in general to a medical laser video endoscope and more particularly to one in which the operating probe may be economically disposed after each use. The scope of protection is defined by the appended claims.

Laser video endoscopes are known and in particular are described in Applicant's issued Patent No. 5,121,740 issued on June 16, 1992 and Patent No. 6,997,868 issued on February 14, 2006, as well as in EP 0 512 592 and in US 6 080 101.

The endoscopes such as the ones described in those two patents, are reused after autoclaving or other sterilization. Reuse occurs in large part because of the expense of the endoscope. The most significant expense factor is the image guide which has a large number of micron size optical fibers. In one endoscope 17,000 fibers were employed thereby providing a 17,000 pixel image.

The image guide currently used costs about $200.00. This is a major incentive for the use of the endoscope after sterilization rather than disposing of the endoscope after each procedure.

This expense factor means that as a practical matter the endoscope will be reused after sterilization rather than disposed of.

However, there is greater security from infection if the probe of the endoscope can be disposed of after each usage instead of being subject to the possibilities of human error in the sterilization process.

Accordingly, it is a key purpose of this invention to provide an endoscope design for which the cost is reasonable enough to permit and encourage disposal of the probe after each use rather then have recourse to sterilization.

It is a related purpose of this invention to provide this cost improvement in a design that maintains a probe design with which the surgeon is familiar and which also maintains the rest of the operating characteristics of the known laser video endoscopes.

It is a further aspect of this invention to provide a laser video endoscope which is less costly than are the current designs.

### Brief Description

A laser video endoscope has a laser guide, an illumination guide and an image guide. These are fiber optical guides which extend through the optical probe and through a hand piece that supports the probe. The hand piece is connected by a first relatively long flexible optical fiber cable to a laser energy source and a source of illumination. A camera assembly is connected to the proximal end of the hand piece and coupled to the optical fiber image guide. A relatively long electrical cable transmits an electrical image signal to a site where an image can be provided for the surgery.

The camera and its electrical cable can be uncoupled from the hand piece and used in a plurality of endoscopic routines.

The rest of the product including the probe and the hand piece can be disposed of after each medical routine thereby providing assurance of an antiseptic procedure.

### Brief Description Of The Drawings

FIG. 1 is a schematic illustration of the prior art system extending from the probe 24 to the terminals 12C, 14C and 16C.

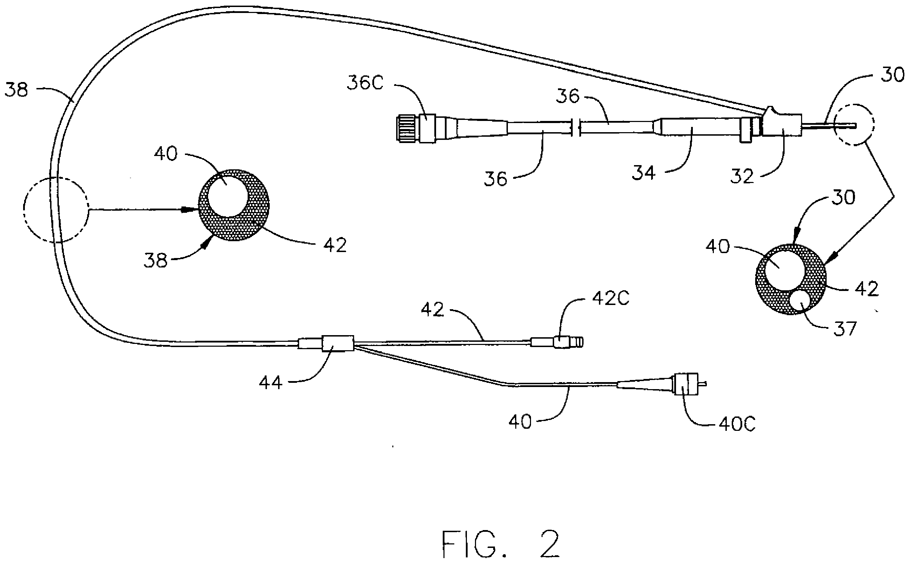

FIG. 2 is a schematic illustration of the embodiment of the invention disclosed herein. FIG. 2, like FIG.1, shows the system extending from the distal probe 30 to proximal terminals 36C, 40C and 42C.

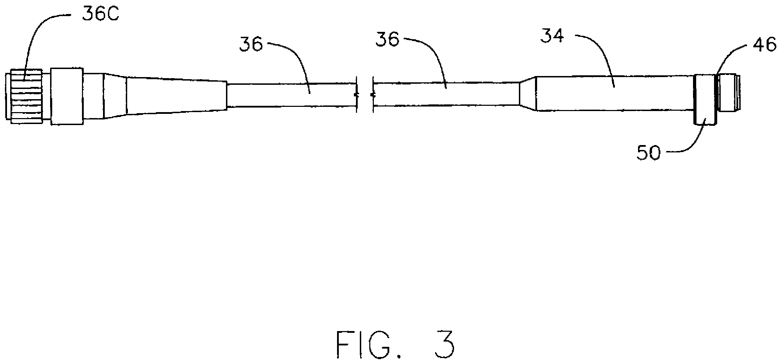

FIG. 3 is a longitudinal view of the camera assembly 34, cable 36 and proximal connector 36C.
FIG. 4 is a partial longitudinal sectional view of the camera assembly 34 showing camera housing 34, focus ring 50 and laser filter 46. FIG. 4 shows the distal recess 52 for engaging the nose 54 of the hand piece 32.
FIG. 5 is partial sectional view of the hand piece 32.
FIG. 6 is a view similar to that of FIGs. 4 and 5 showing the probe 30 and hand piece 32 assembled and coupled to the camera assembly 34.

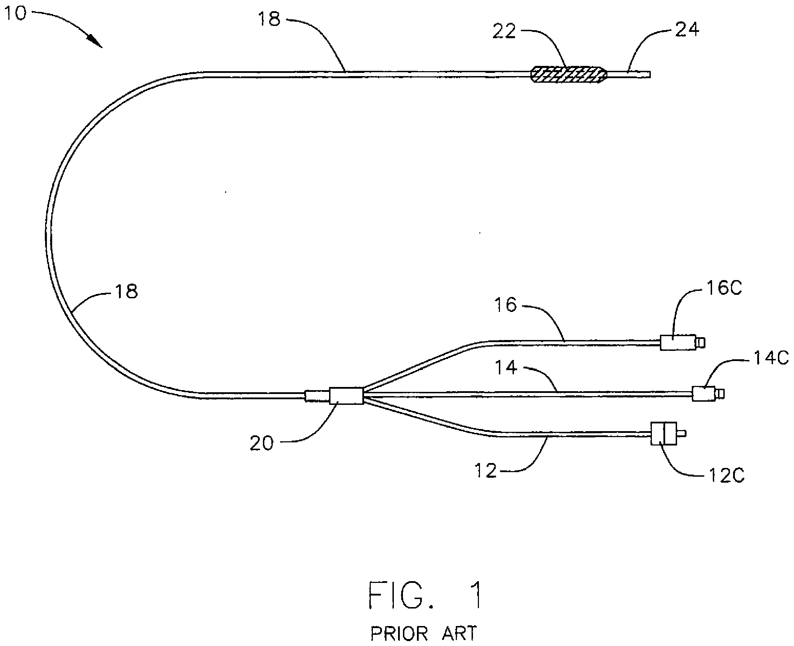

Except for the prior art FIG.1, the figures are all to a single embodiment.

As shown in FIG.1 , the known laser video endoscopes have an operating probe 24, a hand piece 22, a cable 18 which carries a laser guide 12, an illumination guide 14 and an image guide 16. These are all fiber optic guides which extend from the distal end of the probe 24 to the terminals 12C, 14C and 16C. Distal of the trifurcation zone 20, the fiber optic guides are combined geometrically to provide a minimum diameter cable.

The laser video endoscope of this invention includes the probe 30, a specifically designed hand piece 32 and a camera assembly 34 coupled to the proximal end of the hand piece 32.

The camera assembly 34 is directly connected to the proximal end of the hand piece 32. It has a relatively long electrical cable 36 which extends proximally to a terminal 36C which is coupled to an appropriate display mechanism including a video screen so that the operating surgeon can view the image during the course of manipulating the probe.

A optical guide cable 38 extends in the proximal direction from the hand piece 32 to a bifurcation junction 39. This cable 38 carries the laser and illumination guides 40 and 42 for conveying the laser energy and the illumination energy to the probe 30. At the bifurcation junction 44, the laser guide 40 and illumination guide 42 are separated and terminated at the terminals 40C and 42C for connection to the sources of laser energy and illumination energy. The image carrying electrical cable 36 is about as long as is the optical guide cable 38. Each cable 36, 38 can be as long as required for an installation.

As shown by the coupling mechanism in the camera assembly 34, the optical fibers 37 from the probe 30 and hand piece 32, which carry the image are removably coupled to the camera so that the camera provides an electrical image that is transmitted along the electric cable 36 to the terminal 36C at the base where the video displays are provided. The camera may be any one of a number of known type and may be specially designed to fit the geometry of the camera assembly.

Thus by positioning the camera assembly 34 at the hand piece 32, the lengthy and expensive optical image guide is avoided. The camera assembly 34 can be uncoupled from the hand piece 32 so that the relatively expensive camera assembly can be reused. This combination of reuse of the camera assembly 34 and elimination of an extensive length of expensive fiber optic image guide means that disposability of the probe 30 is economically acceptable even though the hand piece 32 and the laser and illumination guides 40, 42 in the cable 38 are also disposed of after each medical routine.

The camera assembly 34 includes a laser filter 46 to protect the camera film from laser energy and to permit the surgeon to observe the operation even when laser pulses are firing. The probe 30 and hand piece 32 are cemented together by a known process.

The camera assembly 34 includes a manually operated spring latch (not shown). The latch is of a known type. It enables readily mounting the camera assembly 34 to the hand piece 32 and, most importantly, removing the camera assembly 34 from the hand piece 32. In addition, the camera assembly 34 includes a focus ring 50 to assure adequate focus of the image provided at the proximal end of the laser fiber image guide 37 in the probe 30 and hand piece 32 onto the image receptors of the camera.

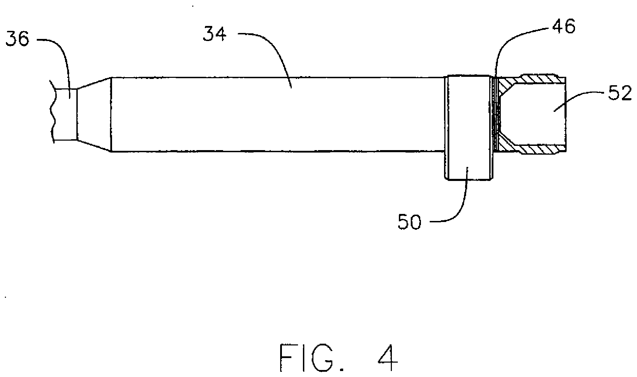

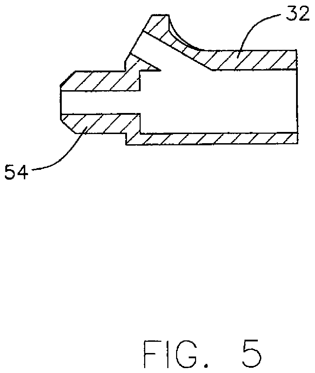

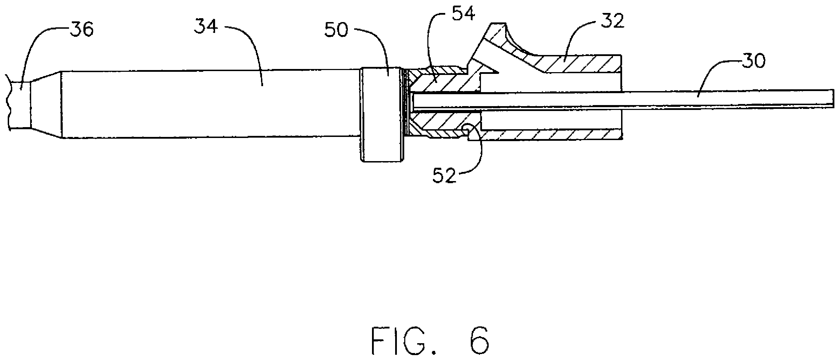

As may be seen from FIGs. 4, 5 and 6, the distal end of the camera assembly has a recess 52 which engages a nose 54 of the hand piece 32. The latch holds the nose 54 in place in the recess 52.

The image guide 37 in the probe 30 and hand piece 32 costs about $8.00. This reduction in cost from about $200.00 to $8.00 is a major factor encouraging disposable use of the endoscope.

A variant on the illustrated embodiment is an arrangement in which the uncoupling at the proximal end of the hand piece 32 will uncouple not only the camera assembly 34 but also the cable 38 so that only the probe 30 and the hand piece 32 would be disposed of between each operation.

It has to be kept in mind that the positioning of the camera assembly 34 at the hand piece 32 permits a standard optical coupling of the image at the proximal end of the optical fiber image guide 37 to the camera assembly 34. It is not feasible to provide a mechanism that will permit coupling and uncoupling the fiber optic image guide 37 at a junction other than the input to the camera. Coupling and uncoupling is otherwise not feasible because of the enormous number of optical fibers which would have to be aligned for such coupling to provide an image that is not degraded or useless.

While the foregoing description and drawings represent the presently preferred embodiments of the invention, it should be understood that those skilled in the art will be able to make changes and modifications to those embodiments without departing from the teachings of the invention and the scope of the claims.

For example, the image guide 37 within the probe 30 and hand piece 32 is a fiber optic bundle of the type normally used. However, there are other means to provide an image guide. One such is the gradient index lens, often referred to as a GRIN lens.

## Claims

1. A surgical endoscope having a hand piece (32), a probe (30) extending distally from said hand piece (32) and rigidly coupled with said hand piece (32), the probe (30) and the hand piece (32) containing an illumination guide (42), a laser guide (40) and an image guide (37), an optical cable (38) containing the illumination guide (42) and the laser guide (40) extending proximally from the hand piece (32) to a laser source and an illumination source, a camera assembly (34) receiving the image from the image guide (37) of the probe (30) to provide an electrical image at the output of the camera assembly (34), and an electrical cable (36) connected to the proximal end of the camera assembly (34) and extending proximally to a terminal (36C) adapted to be connected to a mechanism for displaying the electrical image, **characterized in that**
the hand piece (32) comprises:
a distal end comprising a distal surface and a proximal end comprising first and second proximal surfaces,
a first opening in the first proximal surface;
a second opening in the second proximal surface;
a third opening in the distal surface;
the first opening and the second opening do not overlap with each other;
the probe (30) extends distally from the third opening at the distal surface of the hand piece (32);
the illumination guide (42), the laser guide (40), and the image guide (37) terminate at a distal end of the probe (30) and extend proximally from the distal end of the probe (30) to the proximal end of the probe (30), into the third opening of the hand piece (32);
the illumination guide (42) and the laser guide (40) extend proximally from the third opening of the hand piece (32) to the first opening in the first proximal surface of the hand piece (32);
the image guide (37) extends proximally from the third opening of the hand piece (32) to the second opening in the second proximal surface of the hand piece (32), a proximal end of the image guide (32) terminating at the second proximal surface of the hand piece (32); and
the camera assembly (34) is removably connected directly to the proximal end of said hand piece (32) at the second proximal surface of the hand piece (32) to optically couple the proximal end of the image guide (37) in the hand piece (32) directly to an input of the camera assembly (34), whereby, after each use of the endoscope, said camera assembly (34) can be detached from said hand piece (32), including the illumination guide (42) and the laser guide (40), and the image guide (37), contained in said probe (30) and said hand piece (32).

2. The endoscope of claim 1 wherein: said camera assembly (34) incorporates a laser blocking filter and a focusing mechanism.

3. The endoscope of claim 1 or 2 wherein: said illumination guide (42), said laser guide (40) and said electrical cable (36) each terminate proximally in respective separate terminals (42C, 40C, 36C).

## Patentansprüche

1. Chirurgisches Endoskop mit einem Handstück (32), einer Sonde (30), die sich distal vom Handstück (32) erstreckt und starr mit dem Handstück (32) verbunden ist, wobei die Sonde (30) und das Handstück (32) eine Beleuchtungsführung (42), eine Laserführung (40) und eine Bildführung (37) enthalten, wobei sich ein optisches Kabel (38), das die Beleuchtungsführung (42) und die Laserführung (40) enthält, proximal vom Handstück (32) zu einer Laserquelle und einer Beleuchtungsquelle erstreckt, wobei eine Kameraanordnung (34) das Bild von der Bildführung (37) der Sonde (30) empfängt, um ein elektrisches Bild am Ausgang der Kameraanordnung (34) bereitzustellen, und ein elektrisches Kabel (36), das mit dem proximalen Ende der Kameraanordnung (34) verbunden ist und sich proximal zu einer Anschlussklemme (36C) erstreckt, die zur Verbindung mit einem Mechanismus zum Anzeigen des elektrischen Bilds angepasst ist, **dadurch gekennzeichnet, dass**
das Handstück (32) das Folgende umfasst:
ein distales Ende, das eine distale Fläche umfasst, und ein proximales Ende, das erste und zweite proximale Flächen umfasst;
eine erste Öffnung in der ersten proximalen Fläche;
eine zweite Öffnung in der zweiten proximalen Fläche;
eine dritte Öffnung in der distalen Fläche;
wobei sich die erste Öffnung und die zweite Öffnung nicht überlappen;
sich die Sonde (30) distal von der dritten Öffnung an der distalen Fläche des Handstücks (32) erstreckt;
die Beleuchtungsführung (42), die Laserführung (40) und die Bildführung (37) an einem distalen Ende der Sonde (30) enden und sich proximal vom distalen Ende der Sonde (30) zum proximalen Ende der Sonde (30) in die dritte Öffnung des Handstücks (32) erstrecken;
die Beleuchtungsführung (42) und die Laserführung (40) sich proximal von der dritten Öffnung des Handstücks (32) zur ersten Öffnung in der ersten proximalen Fläche des Handstücks (32) erstrecken;
die Bildführung (37) sich proximal von der dritten Öffnung des Handstücks (32) zur zweiten Öffnung in der zweiten proximalen Fläche des Handstücks (32) erstreckt, wobei ein proximales Ende der Bildführung (32) an der zweiten proximalen Fläche des Handstücks (32) endet; und
die Kameraanordnung (34) entfernbar direkt mit dem proximalen Ende des Handstücks (32) an der zweiten proximalen Fläche des Handstücks (32) verbunden ist, um das proximale Ende der Bildführung (37) im Handstück (32) direkt mit einem Eingang der Kameraanordnung (34) optisch zu verbinden, wodurch nach jeder Anwendung des Endoskops die Kameraanordnung (34) vom Handstück (32) gelöst werden kann, einschließlich der Beleuchtungsführung (42) und der Laserführung (40) und der Bildführung (37), die in der Sonde (30) und im Handstück (32) enthalten sind.

2. Endoskop nach Anspruch 1, wobei: die Kameraanordnung (34) einen Laserblockierfilter und einen Fokussiermechanismus aufweist.

3. Endoskop nach Anspruch 1 oder 2, wobei: die Bildführung (42), die Laserführung (40) und das elektrische Kabel (36) jeweils proximal in jeweiligen getrennten Anschlussklemmen (42C, 40C, 36C) enden.

## Revendications

1. Endoscope chirurgical présentant une pièce à main (32), une sonde (30) qui s'étend de façon distale à partir de ladite pièce à main (32) et qui est couplée de façon rigide à ladite pièce à main (32), la sonde (30) et la pièce à main (32) contenant un guide d'éclairage (42), un guide de laser (40) et un guide d'image (37), un câble optique (38) contenant le guide d'éclairage (42) et le guide de laser (40) s'étendant de façon proximale à partir de la pièce à main (32) jusqu'à une source de laser et une source d'éclairage, un ensemble de caméra (34) qui reçoit l'image en provenance du guide d'image (37) de la sonde (30) afin de produire une image électrique à la sortie de l'ensemble de caméra (34), et un câble électrique (36) qui est connecté à l'extrémité proximale de l'ensemble de caméra (34) et qui s'étend de façon proximale jusqu'à une borne (36C) adaptée pour être connectée à un mécanisme pour afficher l'image électrique, **caractérisé en ce que** la pièce à main (32) comprend:
une extrémité distale présentant une surface distale et une extrémité proximale présentant des première et seconde surfaces proximales,
une première ouverture dans la première surface proximale;
une deuxième ouverture dans la seconde surface proximale;
une troisième ouverture dans la surface distale;
la première ouverture et la deuxième ouverture ne se chevauchent pas mutuellement;
la sonde (30) s'étend de façon distale à partir de la troisième ouverture à la surface distale de la pièce à main (32);
le guide d'éclairage (42), le guide de laser (40) et le guide d'image (37) se terminent à une extrémité distale de la sonde (30) et s'étendent de façon proximale à partir de l'extrémité distale de la sonde (30) jusqu'à l'extrémité proximale de la sonde (30), dans la troisième ouverture de la pièce à main (32);
le guide d'éclairage (42) et le guide de laser (40) s'étendent de façon proximale à partir de la troisième ouverture de la pièce à main (32) jusqu'à la première ouverture dans la première surface proximale de la pièce à main (32);
le guide d'image (37) s'étend de façon proximale à partir de la troisième ouverture de la pièce à main (32) jusqu'à la deuxième ouverture dans la seconde surface proximale de la pièce à main (32), une extrémité proximale du guide d'image (32) se terminant à la seconde surface proximale de la pièce à main (32); et
l'ensemble de caméra (34) est connecté de façon amovible directement à l'extrémité proximale de ladite pièce à main (32) à la seconde surface proximale de la pièce à main (32) afin de coupler de façon optique l'extrémité proximale du guide d'image (37) dans la pièce à main (32) directement à une entrée de l'ensemble de caméra (34), moyennant quoi, après chaque utilisation de l'endoscope, ledit ensemble de caméra (34) peut être détaché de ladite pièce à main (32), comprenant le guide d'éclairage (42) et le guide de laser (40), et le guide d'image (37), contenus dans ladite sonde (30) et dans ladite pièce à main (32).

2. Endoscope selon la revendication 1, dans lequel ledit ensemble de caméra (34) incorpore un filtre de blocage de laser et un mécanisme de focalisation.

3. Endoscope selon la revendication 1 ou 2, dans lequel ledit guide d'éclairage (42), ledit guide de laser (40) et ledit câble électrique (36) se terminent chacun de façon proximale dans des bornes séparées respectives (42C, 40C, 36C).
